# EUROPÄISCHE PATENTSCHRIFT

(11) **EP 2 291 658 B1**
(45) Veröffentlichungstag und Bekanntmachung des Hinweises auf die Patenterteilung: **24.07.2013**
(21) Anmeldenummer: 09769027.5
(22) Anmeldetag: 26.06.2009
(51) Int. Cl.: G01N 33/569, G01N 33/86

(54) **DURCHFLUSSZYTOMETRISCHES VERFAHREN ZUR BESTIMMUNG VON GESAMTLEUKOZYTENZAHL UND TROMBOZYTENZAHL SOWIE ZUR LEUKOZYTENDIFFERENZIERUNG IN BLUTPROBEN VON VÖGELN**
FLOW-CYTOMETRIC METHOD FOR THE DETERMINATION OF THE TOTAL LEUKOCYTE NUMBER AND THROMBOCYTE NUMBER AND FOR THE LEUKOCYTE DIFFERENTIATION IN AVIAN BLOOD SAMPLES
PROCÉDÉ DE CYTOMÉTRIE DE FLUX POUR DÉTERMINER LA NUMÉRATION LEUCOCYTAIRE TOTALE ET LA NUMÉRATION THROMBOCYTAIRE, ET POUR LA DIFFÉRENCIATION DES LEUCOCYTES DANS DES ÉCHANTILLONS SANGUINS D'OISEAUX

(30) Priorität: 27.06.2008 DE 102008030515
(43) Veröffentlichungstag der Anmeldung: 09.03.2011
(73) Patentinhaber: Seliger, Christian, 55118 Mainz (DE)
(72) Erfinder: KOTHLOW, Sonja, 88400 Biberach-Mettenberg (DE); KASPERS, Bernd, 85604 Zorneding (DE); SELIGER,Christian, 55118 Mainz (DE)
(74) Vertreter: Weiss, Wolfgang
(86) Internationale Anmeldenummer: PCT/EP2009/004647
(87) Internationale Veröffentlichungsnummer: WO 2009/156178

(56) Entgegenhaltungen:
- EP-A- 0 552 707
- US-A- 5 047 321
- BURGESS S C ET AL: "Counting absolute numbers of specific leukocyte subpopulations in avian whole blood using a single-step flow cytometric technique: comparison of two inbred lines of chickens" JOURNAL OF IMMUNOLOGICAL METHODS, ELSEVIER SCIENCE PUBLISHERS B.V.,AMSTERDAM, NL, Bd. 227, Nr. 1-2, 30. Juli 1999 (1999-07-30), Seiten 169-176, XP004178052 ISSN: 0022-1759 in der Anmeldung erwähnt
- ANONYMOUS: "Rundbrief", ALUMNI DER MÜNCHNER TIERÄRZTLICHEN FAKULTÄT, no. 15, 2009, pages 18-20,
- ANONYMOUS: "Rundbrief", ALUMNI DER MÜNCHNER TIERÄRZTLICHEN FAKULTÄT, no. 17, 2010, pages 38-39,
- SELIGER CHRISTIAN ET AL: "A rapid high-precision flow cytometry based technique for total white blood cell counting in chickens", VETERINARY IMMUNOLOGY AND IMMUNOPATHOLOGY, vol. 145, no. 1-2, January 2012 (2012-01), pages 86-99, ISSN: 0165-2427

## Beschreibung

Die vorliegende Erfindung betrifft ein Verfahren zur Bestimmung der Gesamtleukozytenzahl und der Thrombozytenzahl sowie zur Leukozytendifferenzierung im Blut von Vögeln unter Verwendung von fluoreszenzmarkierten Panleukozyten- und Thrombozytenmarkern und/oder weiteren Leukozytenmarkern ohne vorherige Abtrennung von Erythrozyten.

Das rote und das weiße Blutbild werden in der Human- und Säugetiermedizin routinemäßig erstellt, um den Gesundheitsstatus von Individuen und Populationen zu überprüfen. Veränderungen in der Homöostase der Leukozyten bilden sich im weißen Blutbild (Leukogramm) ab. Dieses beinhaltet die Bestimmung der Gesamtleukozytenzahl, das Differentialblutbild, die Berechnung der absoluten Zahlen der verschiedenen Leukozytenfraktionen je µl Blut und die morphologische Beurteilung der Leukozyten am gefärbten Blutausstrich.

Das Leukogramm gibt dem Kliniker Einblick in den aktuellen Zustand des Immunsystems von Patienten. Weil Leukozytenzahlen bei gesunden Individuen relativ konstant sind, sich aber beim Vorliegen einer Krankheit möglicherweise stark verändern, hat das weiße Blutbild eine Indikatorfunktion. Leukozytenantworten sind zwar in der Regel nicht pathognomonisch (bereits für sich alleine genommen hinreichend für eine sichere Diagnosestellung). Sie können aber wertvolle Informationen liefern, die dabei helfen, eine Verdachtsdiagnose zu stellen, eine Differentialdiagnose zu bestätigen, den Erfolg einer Therapie zu überwachen oder eine Prognose abzugeben. Veränderungen des weißen Blutbildes ziehen im klinischen Alltag mitunter unmittelbar therapeutische Konsequenzen nach sich, z.B. in Form einer antibiotischen Behandlung. Schließlich sind sie meist einer der ersten labordiagnostischen Hinweise auf das Vorliegen von lebensbedrohlichen Infektionen.

Die Bestimmung der Gesamtleukozytenzahl und des Differentialblutbildes gehören zu den am häufigsten durchgeführten Laboruntersuchungen in der Humanmedizin. Aufgrund der hohen Präzision, Richtigkeit und Schnelligkeit automatischer Hämatologie-Analysegeräte werden diese Untersuchungen in der Humanmedizin mittlerweile überwiegend automatisiert durchgeführt. Nur die Proben, bei deren Analyse das Gerät versagt oder bei denen dieses aufgrund von Besonderheiten eine Überprüfung anfordert, werden mikroskopisch beurteilt.

Im Gegensatz zur Hämatologie bei Säugern befindet sich die Vogelhämatologie immer noch in ihren Anfängen. Die Morphologie der Blutzellen der Vögel wurde beschrieben und es wurden manuelle Techniken zur Leukozytenzählung entwickelt. Entscheidende Fortschritte in der Vogelhämatologie wurden aber verhindert, da automatisierte Untersuchungsverfahren fehlen.

Keines der in der Säugerhämatologie etablierten automatisierten Verfahren ist zur Untersuchung von Vogelblut geeignet. Die Gründe dafür liegen im besonderen Aufbau der Blutzellen der Vögel. Bei der Leukozytenzählung in Hämatologie-Analyse-Geräten werden die störenden Erythrozyten üblicherweise durch Zugabe von Ammoniumchlorid oder hypotonen Lösungen lysiert. Die Erythrozyten der Vögel sind jedoch wie die aller nicht zu den Säugetieren gehörenden Wirbeltiere (Reptilien, Amphibien, Fische, etc.) kernhaltig, lassen sich nur schlecht lysieren und können bei der Messung nicht zuverlässig von Leukozyten unterschieden werden. Zudem handelt es sich bei den Thrombozyten der Vögel, anders als bei den Blutplättchen der Säuger, um lymphozytengroße kernhaltige Zellen, die die Hämatologie-Analyse-Geräte ebenfalls nicht zuverlässig von Leukozyten differenzieren können. In der Literatur wird außerdem darauf hingewiesen, dass neben intakten Erythrozyten und Thrombozyten auch die bei einer Lyse der Erythrozyten freiwerdenden Kerne zu Interferenzen führen. Die gewöhnlichen elektronischen Partikelzählverfahren und die üblichen Zählkammerverfahren können damit nicht zur Ermittlung der Gesamtleukozytenzahl in Vogelblut eingesetzt werden.

Deshalb wurden zahlreiche manuelle Verfahren zur Bestimmung der Gesamtleukozytenzahl in Vogelblut entwickelt. Alle diese Techniken sind jedoch zeitaufwändig und ungenau. Die niedrige Anzahl gezählter Zellen bei den mikroskopischen Verfahren resultiert in einem hohen statistischen Fehler. Die zusätzlichen Schwierigkeiten bei der Leukozytendifferenzierung beim Vogelblut führen zu Verwechslungen und machen die mikroskopischen Verfahren noch unzuverlässiger und fehleranfälliger.

Versuche, die verfügbaren Geräte für die Leukozytenzählung und -differenzierung von Vogelblut zu adaptieren, verliefen überwiegend erfolglos. Zwar wird von zufriedenstellenden Ergebnissen beim Einsatz des Hämatologie-Analyse-Gerätes Cell-Dyn 3500 der Firma Abbott Laboratories zur Untersuchung des Blutes von Papageien, Sperlingsvögeln, Wasservögeln und Laufvögeln berichtet (Fudge, Abstract in Main Conference Proceedings der Association of Avian Veterinarians 1995). Das Abstract endet mit dem Hinweis, dass in der Präsentation vorläufige Präzisionsdaten, die publiziert werden sollen, erörtert würden. Bis zum gegenwärtigen Zeitpunkt konnte allerdings keine weitere Informationen enthaltende Publikation des Autors zu diesem Thema gefunden werden. In einem fünf Jahre später von ihm herausgegebenen Buch schreibt er, die Technologie der Durchflusszytometrie bedürfe noch der Verbesserung. Auf die Probleme eingehend erklärt er, es seien für die verschiedenen Vogelspezies aufgrund ihrer unterschiedlichen Leukozytenmorphologie verschiedene Geräteeinstellungen erforderlich. Außerdem werden Schwierigkeiten bei der Unterscheidung zwischen Thrombozyten und Lymphozyten eingestanden.

Eine andere Autorin berichtet gar, dass mit dem Cell-Dyn-3500-Gerät eine Differenzierung der Leukozyten in Lymphozyten und Granulozyten nicht gut möglich war (Vergleich hämatologischer Untersuchungsmethoden bei Vögeln. Diss, Vet. Med. Univ. Wien Reauz, E. 1996). Lediglich bei der Messung des Hämatokrits und der Gesamtleukozytenzahl sei ein befriedigendes Ergebnis erzielt worden. In ihrer Arbeit hat Reauz Vogelblut mit noch einem weiteren Hämatologie-Automaten (MS9, Melet Schloesing, Frankreich) untersucht. Dabei seien für die Parameter Hämatokrit, Erythrozytenzahl, Gesamtleukozytenzahl, Lymphozyten, Monozyten und Granulozyten Werte erzielt worden, die gut mit den mit Standardmethoden ermittelten Ergebnissen übereinstimmten. Darüber hinausgehende Publikationen über den Einsatz dieses Gerätes zur Untersuchung von Vogelblut konnten allerdings nicht gefunden werden.

Eine weitere Untersuchung von Hühnerblut mit dem Cell-Dyn 3500 berichtet von Granulozytenwerten, die eine "annehmbare" Genauigkeit aufweisen. Trotz des Einsatzes der vom Hersteller angebotenen Spezialsoftware VET 2.3 mit besonderen Einstellungen für die Untersuchung von Vogelblut seien die Lymphozytenzahlen jedoch vollkommen ungenau gewesen, zumal bei Mehrfachmessungen der gleichen Proben zum Teil beachtliche Differenzen zwischen den Messergebnissen zu verzeichnen gewesen seien. Deshalb hat der Autor wenig Hoffnung, dass die automatisierte Untersuchung von Vogelblut ähnlich gut funktionieren kann wie die bei den Säugern.

Die zuerst in der Humanmedizin eingesetzte Quantitative Buffy Coat-Technologie ermöglicht auch bei verschiedenen Haussäugetieren eine schnelle ungefähre Bestimmung der Gesamtleukozytenzahl sowie die Erstellung eines Differentialblutbildes. Seidl teste das Verfahren an Blutproben von Psittaziden. Der einzige Parameter, der an Vogelblut zuverlässig bestimmt werden konnte, war jedoch der Hämatokrit. Als Ursache für die nicht zufrieden stellenden Ergebnisse bei den anderen Parametern wird die nicht exakte Auftrennung der einzelnen Zellbanden angegeben.

Angesichts der bisher nicht gelösten Schwierigkeiten bei der automatisierten Untersuchung von Vogelblut wird bislang also in der Vogelmedizin immer noch weitgehend auf arbeitsaufwändige und unzuverlässige mikroskopische Verfahren zurückgegriffen oder ganz auf diese wichtigen hämatologischen Untersuchungen verzichtet.

Deshalb besteht weiterer Forschungsbedarf ein automatisiertes Verfahren zu entwickeln, das diese Probleme überwindet. Ein solches Verfahren würde die Erforschung der Vogelhämatologie beflügeln.

Es besteht seit langem ein Bedarf an der Untersuchung größerer Zahlen von Geflügelblutproben, der aber aufgrund des Fehlens einer automatisierten Methode zur Erstellung eines weißen Blutbildes beim Vogel bisher nicht befriedigt werden kann.

In wissenschaftlichen Untersuchungen wird die Bestimmung des Verhältnisses zwischen Heterophilen Granulozyten und Lymphozyten vielfach als Maß für die Stressbelastung von Hühnern genutzt. Zudem findet die Bestimmung des Heterophilen Granulozyten/Lymphozyten-Verhältnisses bereits Anwendung bei der Zucht stress- bzw. krankheitsresistenter Hühnerlinien. Auch im Rahmen der Entwicklung und Prüfung von Geflügelimpfstoffen besteht Bedarf für die Erstellung weißer Blutbilder. So kommen beim Geflügel verschiedene immunsuppressive Viruskrankheiten vor, die enorme wirtschaftliche Schäden verursachen. Im Rahmen von Studien zur Sicherheit von Lebend-Impfstoffen gegen solche Krankheiten wurde bereits von verschieden Impfstoff-Herstellern gegenüber universitären Forschungseinrichtungen Interesse signalisiert, größere Anzahlen von weißen Blutbildern bei Hühnern erstellen zu lassen. Schließlich fordert die EU-Richtlinie 2001/82/EG (Abschnitt C 5) für die Zulassungsverfahren von Tierimpfstoffen in den Mitgliedsstaaten: "Sofern das immunologische Tierarzneimittel negative Auswirkung auf die Immunreaktion des geimpften Tieres oder seiner Nachkommen haben könnte, sind angemessene Versuche über die immunologischen Funktionen durchzuführen". Welche Bedeutung dem Komplex der Immunsuppressiven Krankheiten beim Geflügel europaweit beigemessen wird, zeigt auch die durch die EU finanzierte COST Action 839 "Immunosuppressive Viral Diseases in Poultry".

In der zellbiologischen Forschung hat sich mit der Durchflusszytometrie eine äußerst leistungsfähige Technik zur Differenzierung und Zählung verschiedenster in Suspension vorliegender Zellen etabliert.

Die Technik wird bereits in der humanmedizinischen Routinediagnostik eingesetzt. Klassisches Beispiel dafür ist die Zählung der CD4-positiven T-Zellen im Rahmen der AIDS-Diagnostik und für Verlaufskontrollen bei HIV-Patienten. Ähnliche Anwendungen der Durchflusszytometrie sind die Zählung von Monozyten und hämatopoetischen Zellen.

Obwohl seit Jahren das Problem bekannt und der Bedarf vorhanden war, ist nicht dokumentiert, dass mit Hilfe der Durchflusszytometrie ein praxistaugliches automatisiertes Verfahren entwickelt werden konnte, welches eine Bestimmung der Gesamtleukozytenzahl sowie die Erstellung eines Differentialblutbildes beim Huhn oder anderen Vögeln ermöglicht.

Es wurde zwar bereits ein durchflusszytometrisches Verfahren zur Bestimmung der Lymphozytenzahl im Blut von Wachteln publiziert, das auf der Verwendung der Farbstoffe 3,3'-Dipentyloxacarbocyaninjodid (DiOC₅(3)) oder 3,3'-Dihexyloxacarbocyaninjodid (DiOC₆(3)) beruht (Moritomo et al., J. Vet. Med. Sci. 64 (2002), 1149-1151; Uchiyama et al., J. Vet. Med. Sci. 67 (2005), 441-444). Diese Technik hat sich bis zum gegenwärtigen Zeitpunkt aber weder in der Forschung noch in der Routinediagnostik durchgesetzt. Eigene Untersuchungen zeigen, dass mit dieser Technik schon bei der Analyse von Hühnerblut oftmals keine zuverlässige Unterscheidung der verschiedenen Zellpopulationen gelingt.

Andere durchflusszytometrische Techniken werden in der Wissenschaft auch von verschiedenen Arbeitsgruppen, die das Immunsystem des Huhns erforschen, zur Identifizierung und Zählung unterschiedlicher Leukozytenfraktionen eingesetzt.

In Forschungsarbeiten zur Hühnerimmunologie geht der Untersuchung von Leukozytensubpopulationen in der Regel die Isolierung der Leukozyten mit Hilfe spezieller Zentrifugationstechniken voraus, um sie von den störenden nicht lysierbaren Erythrozyten abzutrennen (siehe z.B. Bohls et al., Dev. Comp. Immunol.). Auf diese Weise aufbereitete Proben erlauben jedoch keine absolute Quantifizierung der betreffenden Zellen bezogen auf das Blutvolumen. Zudem enthalten solche üblicherweise mit Hilfe der "Slow speed"- oder der "Ficoll-Dichte-Gradienten"-Zentrifugation hergestellten Zellpräparationen keine oder nur sehr wenige Granulozyten, da diese zusammen mit den Erythrozyten abgetrennt werden.

In der Literatur ist eine Methode zur durchflusszytometrischen Zählung von Leukozyten-Subpopulationen in Vollblutproben von Hühnern beschrieben (Burgess und Davison, J. Immunol. Meth. 227 (1999), 169-176). Die Technik basiert im Wesentlichen auf dem beispielsweise in der Humanmedizin bei der AIDS-Diagnostik und AIDS-Therapieüberwachung routinemäßig für die Quantifizierung der CD4 positiven T-Helferzellen eingesetzten Verfahren. Es werden dabei direkt mit Fluoreszenzfarbstoffen gekoppelte monoklonale Antikörper eingesetzt. Um eine Zellkonzentration zu erhalten, die vom Durchflusszytometer noch verarbeitet werden kann, wird die Blutprobe vor der Messung stark verdünnt. Folglich muss die Messdauer auf ein Vielfaches erhöht werden, um dennoch eine ausreichende Anzahl an Leukozyten erfassen zu können, da es sich ja bei der überwiegenden Mehrzahl der in der Probe vorhandenen Zellen um Erythrozyten handelt. Zusätzlich werden fluoreszierende Partikel in bekannter Konzentration zugegeben, um absolute Zellzahlen ermitteln zu können.

Burgess et al. erkannten zwar, dass mit der von ihnen beschriebenen Technik spezifische Populationen von PBLs (Peripheren Blut Lymphozyten) identifiziert und quantifiziert werden können. Ein Hinweis, das Verfahren zur Bestimmung der Gesamtleukozytenzahl oder zur Erstellung eines vollständigen Differentialblutbildes nutzen zu können, findet sich jedoch nicht.

Gemäß der vorliegenden Erfindung wird durch den kombinierten Einsatz eines Panleukozytenmarkers und eines Thrombozytenmarkers auch bei der direkten Färbung von Vollblut eine sichere Abgrenzung der Populationen Erythrozyten, Thrombozyten und Leukozyten ermöglicht.

Ein Gegenstand der Erfindung ist somit ein Verfahren zur Bestimmung der Gesamtleukozytenzahl im Blut von Vögeln oder Hühnervögeln, umfassend die Schritte:
(a) Bereitstellung einer nicht-koagulierenden Blutprobe,
(b) gegebenenfalls Fixieren der Probe,
(c) Verdünnen der Probe,
(d) Inkontaktbringen der verdünnten Probe mit einem Panleukozytenmarker und einem Thrombozytenmarker,
(e) Messen der Probe in einem Durchflusszytometer ohne vorherige Wasch- oder Aufreinigungsschritte und
(f) quantitatives Bestimmen der Leukozytenpopulation sowie der Thrombozytenpopulation in der Probe gemäss Anspruch 1.

Bevorzugte Ausführungen sind in den Ansprüchen 2-6 beschrieben. Ausführungen in der Beschreibung, die sich nicht auf die Bestimmung der Gesamtleukozytenzahl im Blut von Vögeln oder Hühnervögeln beziehen, sind lediglich als Referenzbeispiele genannt.

Das Verfahren gestattet die Bestimmung von Gesamtleukozytenzahl und Thrombozytenzahl sowie die Erstellung von Differentialblutbildern (Leukozytendifferenzierung) beim Huhn. Die Technik ist bei Verfügbarkeit der entsprechenden Markersubstanzen (monoklonalen Antikörper) übertragbar auf alle anderen Vogelspezies deren Blut ebenfalls aufgrund kernhaltiger Erythrozyten sowie großer Thrombozyten nicht mit den herkömmlichen in der Human- und Säugetiermedizin verbreiteten Verfahren analysiert werden kann.

Das in dieser Beschreibung beschriebene Verfahren bietet auch bei der Untersuchung des Blutes von Spezies, deren kernlose Erythrozyten gut zu lysieren sind, z.B. beim Menschen und anderen Säugetieren, Vorteile gegenüber den in diesen Bereichen üblichen Methoden. Zwar wurden bereits durchflusszytometrische Verfahren zur Differentialblutbilderstellung bei Mensch und Maus publiziert. Diese beinhalten aber im Gegensatz zu der hier beschriebenen Methode in der Regel eine Erythrozytenlyse. Bei den etablierten Lyseverfahren wird auch ein Teil der weißen Blutkörperchen zerstört, so dass die Ergebnisse der anschließend durchgeführten Zellzählung nicht mehr der Realität entsprechen. Zudem kommt es auch bei Säugerblut vor, dass kernhaltige Erythrozyten oder kernhaltige Erythrozytenvorläufer durch die routinemäßig eingesetzten Lyseverfahren nicht aufgelöst werden und anschließend die Zählung stören. Zwar wurde die No-Lyse No-Wash-Technik für die CD4⁺-T-Zell- und CD34⁺-Zell-Zählung beschrieben. Ein Verfahren, das eine Bestimmung der Gesamtleukozytenzahl, Thrombozytenzahl bzw. Differentialblutbilderstellung mit einem Pan-Leukozytenmarker und/oder einem Thrombozytenmarker ohne Wasch- und Aufreinigungsschritte, insbesondere ohne Lyse von Erythrozyten beinhaltet, wurde bisher aber für keine Spezies publiziert.

Aufgrund der beschriebenen Vorteile birgt die beschriebene Technik das Potential als Referenzverfahren für die verschiedensten Spezies eingesetzt zu werden.

In einer besonders bevorzugten Ausführungsform umfasst das Verfahren eine Kombination folgender Schritte:
a) Durchführung in einem einzigen Reaktionsgefäß (Single Tube),
b) Verfahrensführung ohne Lyse von Zellpopulationen, z.B. Erythrozyten (No Lyse),
c) Verfahrensführung ohne Waschschritte an der Probe (No Wash),
d) Gesamtleukozytenzahlbestimmung und
e) Leukozytendifferenzierung und
f) Thrombozytenzahlbestimmung.

Die hier detailliert beschriebene Variante des neuen Verfahrens zur Blutbilderstellung bei Hühnervögeln, insbesondere beim Huhn, ermöglicht die Erstellung eines Differentialblutbildes mit einem Durchflusszytometer mit nur einem Laser und 3 Fluoreszenzkanälen, wie z.B. dem FACScan von Becton Dickinson. Es gestattet damit die Nutzung älterer bzw. kostengünstiger Geräte.

Schritt (a) des erfindungsgemäßen Verfahrens umfasst das Bereitstellung einer nicht-koagulierenden Blut probe, insbesondere einer Vollblutprobe Die Probe kann auf übliche Weise aus dem zu untersuchenden Organismus gewonnen werden. Falls erforderlich, wird die Probe mit einem geeigneten Antikoagulationsreagenz behandelt. Ein bevorzugtes Reagenz ist EDTA oder ein Salz davon. Dabei wurde gefunden, dass insbesondere bei weiblichen adulten Vögeln, wie etwa dem legereifen Huhn, höhere EDTA-Konzentrationen für eine ausreichende Antikoagulationswirkung erforderlich sind. Bevorzugt werden daher ≥ 4 mg/ml EDTA, besonders bevorzugt 4-7 mg/ml EDTA zur Antikoagulation verwendet.

Schritt (b) des erfindungsgemäßen Verfahrens umfasst das Fixieren der Probe. Dieser Schritt ist nur fakultativ und kann bei einer zeitnahen Aufbereitung und Messung der Probe unterbleiben. Die Fixierung der Probe kann mit üblichen Reagenzien, z.B. Paraformaldehyd, Formaldehyd oder kommerziell erhältlichen Fixierungsmitteln, erfolgen.

Schritt (c) des Verfahrens umfasst das Verdünnen der Probe, das günstigerweise im Wesentlichen ohne Erythrozytenlyse durchgeführt wird. Zum Verdünnen wird üblicherweise ein geeigneter Puffer verwendet. Die Verdünnung kann beispielsweise in einem Verhältnis vom 1:1 (ein Volumenteil Probe zu einem Volumenteil Puffer) bis 1:1000, besonders bevorzugt 1:10 bis 1:100, beispielsweise etwa 1:50, erfolgen.

Gemäß Schritt (d) wird die verdünnte Probe mit einem Panleukozytenmarker und einem Thrombozytenmarker in Kontakt gebracht. Dabei handelt es sich üblicherweise um direktmarkierte, z.B. mit Fluoreszenzfarbstoffen markierte Antikörper, die gegen Zelloberflächenantigene gerichtet sind, die für Leukozyten bzw. Thrombozyten charakteristisch sind. Die Antikörper können polyklonale oder monoklonale Antikörper sein. Monoklonale Antikörper sind bevorzugt.

Beispiele für Antikörper, die als Panleukozytenmarker geeignet sind, sind die Antikörper anti-CD45 (Klon 16-6, Klon LT40) und K55. Beispiele für Antikörper, die als Thrombozytenmarker geeignet sind, sind K1, anti-CD41/D61 und anti-CD51/CD61.

Das Inkontaktbringen der Probe mit den Markern erfolgt unter Bedingungen, bei denen die Marker an die betreffenden Zellen binden können.

Anschließend wird die Probe gemäß Schritt (e) in einem Durchflusszytometer gemessen. Das erfindungsgemäße Verfahren zeichnet sich dadurch aus, dass die Probe ohne vorherige Wasch- oder Aufreinigungsschritte, z.B. Abtrennung von Erythrozyten bzw. Lyse von Erythrozyten, durchflusszytometrisch untersucht wird. Dabei erfolgt gemäß Schritt (f) eine quantitative Bestimmung der Leukozytenpopulation, d.h. die Bestimmung der Gesamtleukozytenzahl sowie gegebenenfalls der Thrombozytenpopulation in der Probe. Eine quantitative Bestimmung der Zellen kann durch Einsatz von Quantifizierungsbeads, die in einer vorbestimmten Anzahl der Probe zugegeben werden, und/oder über Flussratenkalibrierung und/oder über die Verwendung eines Durchflusszytometers mit der Möglichkeit zum True Volumetric Counting erzielt werden. Die Daten werden mittels einer geeigneten Software ausgewertet und die verschiedenen Zellpopulationen bestimmt, wobei die absoluten und die relativen Zellzahlen bestimmt werden.

Vorzugsweise umfasst die Bestimmung die Verwendung eines Panleukozytenmarkers und eines Thrombozytenmarkers mit unterschiedlichen Fluoreszenzmarkierungen, wodurch eine Unterscheidung von Thrombozyten und Leukozyten in Gegenwart von Erythrozyten möglich ist. Weiterhin wird günstigerweise eine Vorwärts- (FCS) und Seitwärts-(SSC)-Streuungs/Beugungsanalyse der Zellen durchgeführt, wobei die Monozyten und auch die heterophilen Granulozyten als eindeutig abgrenzbare Populationen bei der Darstellung aller Leukozyten identifiziert werden können.

Durch das erfindungsgemäße Verfahren wird bei einer direkten Färbung von Vollblutproben eine sichere Abgrenzung der Leukozyten-, Thrombozyten- und Erythrozytenpopulationen ermöglicht.

Die weitere Differenzierung der verschiedenen Leukozytenfraktionen kann erfolgen, sobald sich diese in mindestens einem durchflusszytometrisch erfassten Parameter voneinander unterscheiden.

Mit dem Verfahren lassen sich grundsätzlich alle Zellen identifizieren und quantifizieren, für die ein spezifischer Marker oder eine spezifische Markerkombination verfügbar sind. Ist nur ein unspezifischer Marker vorhanden, kann gegebenenfalls in Kombination mit den anderen Eigenschaften Zellgröße (FSC-H) und Granularität (SSC-H) die betreffende Zellpopulation dargestellt werden. So sind z.B. Granulozyten wie alle Leukozyten positiv für den Marker αCD45. Durch ihre starke Granularität (SSC-H) lassen sie sich von diesen aber unterscheiden.

Bereits die Kombination eines Thrombozytenmarkers und eines Panleukozytenmarkers erlaubt eine weitreichende Differenzierung in Erythrozyten, Thrombozyten, Leukozyten, Lymphozyten, Monozyten und Heterophile Granulozyten.

Der modulare Aufbau der Technik gestattet aber eine weitere und zuverlässigere Differenzierung durch den Einsatz zusätzlicher Leukozytenmarker.

Beispiele von Leukozytensubpopulationen, die durch das erfindungsgemäße Verfahren bestimmt werden können, sind T-Helferzellen, cytotoxische T-Zellen, yδ-T-Zellen, NK-Zellen, Basophile Granulozyten, Eosinophile Granulozyten, Heterophile Granulozyten, B-Zellen oder Kombinationen von zwei oder mehreren der genannten Populationen.

Als hervorragend geeignet zur Blutbilderstellung beim Huhn hat sich die in Tabelle 1 aufgeführte Kombination folgender Antikörper erwiesen:

**Tabelle 1**

| **Zielantigen** | **Klon** | **Fluorochrom** | **Gefärbte Zellen** |
|---|---|---|---|
| CD45 | 16-6 | PerCP | Leukozyten, Thrombozyten |
| | kommerziell erhältlich bei Serotec (Division of MorphoSys) | | |
| | K1 | RPE | Thrombozyten, Monozyten |
| | KUL01 | RPE | Monozyten |
| CD4 | CT4 | FITC | T-Helferzellen |
| CD8α | CT8 | FITC | cytotoxische T-Zellen |
| TCRγδ | TCR1 | FITC | γδ-T-Lymphozyten |
| BU1 | AV20 | FITC | B-Lymphozyten |

Das erfindungsgemäße Verfahren ist für Anwendungen in der Human- und Veterinärmedizin geeignet. Insbesondere eignet sich das Verfahren zur Bestimmung eines weißen Blutbildes bei Vögeln, vorzugsweise bei Hühnervögeln und besonders bevorzugt bei Hühnern.

Weiterhin soll das erfindungsgemäße Verfahren durch die nachfolgenden Beispiele erläutert werden.

### Beispiel 1: Gewinnung von Blutproben

Für die Ungerinnbarmachung der Blutproben können verschiedene Antikoagulantien eingesetzt werden. Die Verwendung flüssiger Antikoagulantien ermöglicht eine schnellere Durchmischung mit dem Blut, führt aber zu einer Verdünnung der Probe.

EDTA, z.B. in Form von K₂EDTA oder Na₂EDTA, gilt auch als das Antikoagulans der Wahl für die hämatologische Untersuchung von Vögeln. Es erlaubt eine einwandfreie Anfärbung der Zellen und führt nicht zur Verklumpung von Leukozyten. Bei der Verwendung von Röhrchen, die EDTA in sprühgetrockneter Form beinhalten, kommt es zu keiner Verdünnung der Probe und in der Folge auch zu keiner Verfälschung der absoluten Zellzahlen. EDTA-Konzentrationen zwischen 1,0 und 2,0 mg EDTA/ml Blut gelten bei Säugern und Vögeln als empfehlenswert. Von höheren Konzentrationen wird abgeraten, da diese zur Schrumpfung der Erythrozyten führen können. Bei EDTA-Konzentrationen über 3,75 mg/ml wurde eine starke Blaufärbung des Blutausstriches und Veränderungen der Leukozyten beobachtet.

In eigenen Untersuchungen wurde festgestellt, dass Blut von adulten Legehennen, das in für die Humanmedizin produzierte mit EDTA präparierte Gefäße entnommen wird, oftmals gerinnt. Vorzugsweise werden daher höhere EDTA-Konzentrationen zwischen 4 und 7 mg/ml verwendet.

Dafür bietet sich folgende Vorgehensweise an:
Es werden 1,5 ml Blut durch eine Kanüle in eine 2,0 ml Spritze entnommen. Dann wird das Blut sofort in ein evakuiertes Blutentnahmeröhrchen (z.B. Vacutainer® 7,2 mg K₂EDTA Vakuum 4,0 ml) überführt. Dazu wird mit der immer noch auf der Spritze befindlichen Kanüle die Gummikappe des Röhrchens durchstochen. Auf diese Weise wird das Blut durch das Vakuum von selbst in das Röhrchen eingesaugt.

Alternativ bietet sich die Modifikation kommerziell erhältlicher humanmedizinischer Röhrchen an. Dazu werden z.B. mit einer Spritze, auf die eine Kanüle aufgesetzt ist, 2,5 ml Luft in ein 4,0 ml Röhrchen eingebracht. Bei der Blutentnahme muss dann lediglich darauf geachtet werden, dass mindestens 1,5 ml Blut entnommen werden. Beim Transfer der Blutprobe in das Vakuumröhrchen sorgt dann das reduzierte Vakuum für die korrekte Befüllung des Röhrchens.

Durch Invertieren wird das Blut mit dem Antikoagulans gemischt, um die Bildung von Gerinnseln zu vermeiden. Anschließend werden die Proben nach Möglichkeit bis zur weiteren Verarbeitung bei Raumtemperatur auf einen Taumel-Rollenmischer gelegt, um eine kontinuierliche und schonende Durchmischung zu gewährleisten.

Das hier beschriebene durchflusszytometrische Verfahren ist kompatibel mit der vorherigen Fixierung des Hühnerblutes. Es können fixierende Substanzen wie Paraformaldehyd, Formaldehyd oder kommerziell erhältliche Fixierungsmittel zur Konservierung von Blutproben für durchflusszytometrische Untersuchungen wie TransFix (UKNEQAS), Cyto-Chex BCT (Streck Laboratories), Streck Cell Preservative (Streck Laboratories), Cellsave (Immunocon) oder ThromboFix (Beckmann Coulter) eingesetzt werden. In eigenen Studien konnten Hühnerblutproben nach Fixierung mit TransFix (UKNEQAS) nach 3 Tagen noch zuverlässig analysiert werden. Somit erlaubt das Verfahren eine zentrale Untersuchung bzw. die Einsendung von Proben an ein Untersuchungslabor.

Bei der Fixierung mit TransFix® (UKNEQAS) kann folgendermaßen verfahren werden: Das Blutentnahme-Röhrchen mit der darin enthaltenen Blutprobe wird invertiert, um die Zellen vollständig zu resuspendieren. Dann werden 400 µl EDTA-Blut aus dem Blutentnahme-Röhrchen in ein 0,5 ml Reaktionsgefäß überführt. Daraufhin werden 80 µl Transfix® zu dem Blut in das 0,5 ml Reaktionsgefäß gegeben. Zuletzt wird das Reaktionsgefäß sorgfältig verschlossen und die Probe vorsichtig gemischt. Die Lagerung bis zur Messung erfolgt bei Raumtemperatur.

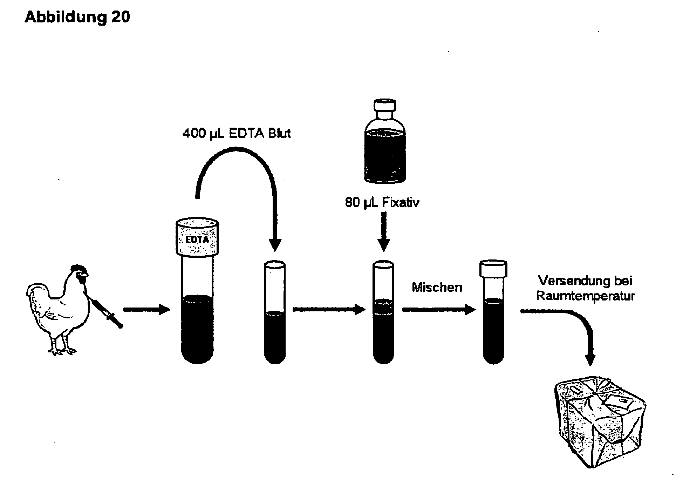

Abbildung 20 zeigt eine schematische Darstellung einer Ausführungsform zur erfindungsgemäßen Fixierung von Hühnerblutproben.

### Beispiel 2: Verdünnen und Anfärben von Proben

Für jeden eingesetzten Antikörper wird in einem Titrationsexperiment die optimale Konzentration bestimmt. Auf diese Weise wird das unspezifische Hintergrundrauschen so reduziert, dass ganz auf Waschschritte verzichtet werden kann.

Üblicherweise wird für eine Färbung 1 µl Blut eingesetzt. Dazu wird das Blut zunächst 1/50 in einem geeigneten Puffer verdünnt und dann 50 µl dieser Vormischung vorgelegt. Die Konzentrationen der eingesetzten Antikörperlösungen werden so gewählt, dass die optimale Konzentration erst im Gesamtvolumen aus Antikörperlösung und vorverdünnter Probe erreicht wird. Die direktkonjugierten Antikörper lassen sich als Vormischung zur verdünnten Blutprobe hinzugeben (Instant-Methode). Alternativ - insbesondere beim Einsatz verschiedener Färbungen - können die Antikörper aber auch einzeln zur Probe gegeben werden. Um die Verluste durch Adhärenz von Zellen (v.a. Thrombozyten, Monozyten, Granulozyten) zu verringern, wird die Färbung unfixierter Proben vorzugsweise in Polypropylenröhrchen durchgeführt.

Um ausgefallenes, bei durchflusszytomeztischen Langzeitmessungen störendes Bovines Serum Albumin (BSA) zu entfernen, wird bei Verwendung eines BSA-haltigen Puffers dieser vorab filtriert.

Vor Beginn der Färbung kann eine, die verschiedenen direkt konjugierten, monoklonalen Antikörper enthaltende Lösung frisch hergestellt werden. Um jeden Antikörper in der optimalen Konzentration einsetzen zu können und zugleich möglichst wenig Reagenzien zu verschwenden, wird zunächst von jedem Antikörper eine vorverdünnte Stammlösung hergestellt. Aus gleichen Anteilen aller Stammlösungen wird dann die zur Färbung eingesetzte Lösung angemischt. Die Antikörper-Konzentrationen der Stammlösungen und der Misch-Lösung werden so berechnet, dass bei der späteren Färbung in dem Gemisch aus Probe und Misch-Lösung die AK-Konzentrationen den in Titrationsexpermenten ermittelten optimalen Konzentrationen entsprechen. Alternativ zur Anmischung unmittelbar vor dem Experiment kann die Antikörpermischung vorher hergestellt und mit einem Konservierungsmittel (z.B. Natriumazid, Pentachlorphenol) versehen und dann über längere Zeiträume gekühlt aufbewahrt werden.

Nach Resuspension werden 20 µl fixiertes EDTA-Blut in ein 1,5 ml Reaktionsgefäß überführt. Zur Herstellung einer 1/50-Verdünnung werden 980 µl sterilfiltrierter Puffer (Raumtemperatur) zu dem Blut in das 1,5 ml Reaktionsgefäß gegeben. Durch Invertieren werden Blut und Puffer sorgfältig gemischt.

| **Fluo-Puffer** | |
|---|---|
| 5 g | Bovines Serum Albumin *(BSA)* |
| 50 mg | Natrium-Azid (NaN₃) |
| ad 500 ml | PBS |
| Lagerung: | 4 °C |

Um die absolute Quantifizierung der identifizierten Zellpopulationen zu ermöglichen wird vorzugsweise nach dem "add-only" Prinzip verfahren. Es werden soweit möglich nur Puffer und Lösungen in das Probenröhrchen zugegeben und bis zur Messung nichts mehr aus dem Röhrchen entnommen bzw. in andere Gefäße überführt.

Sofern zur absoluten Quantifizierung TruCOUNT Tubes und das FACScan-Zytometer (Becton Dickinson) eingesetzt werden empfiehlt sich folgendes Vorgehen:
Die benötigte Menge an TruCOUNT Tubes wird unmittelbar vor der Färbung aus dem bei Raumtemperatur gelagerten Beutel entnommen. Dann werden 20 µl der Antikörpervormischung knapp oberhalb des Metallplättchens an die Seitenwand des TruCOUNT Tube pipettiert, ohne das bead-Kügelchen zu berühren. Als nächstes wird das TruCOUNT Tube um etwa 180 ° um seine Längsachse gedreht. Mittels Reverse Pipetting werden 50 µl der in Fluopuffer verdünnten Blutprobe ebenfalls oberhalb des Metallplätchens an die gegenüberliegende Seitenwand des TruCOUNT Tube pipettiert, ohne das bead-Kügelchen oder den bereits zugegebenen Tropfen der Antikörperlösung zu berühren. Anschließend wird das TruCOUNT Tube drei mal eine Sekunde vorsichtig gevortext. Die Inkubation erfolgt für eine Dauer von ca. 45 Minuten im Dunkeln und bei Raumtemperatur. Dann werden 300 µl Puffer zugegeben und das TruCOUNT Tube in Eis gesteckt. Der Eisbehälter wird zugedeckt um die Proben bis zur Messung gegen Lichteinfall zu schützen.

Alternativ zu den TruCOUNT Tubes können andere Beads (CALTAG Counting Beadsvon Molecular Probes, Flow-Count Fluospheres von Beckman Coulter, CytoCount von DakoCytomation, CountBright von Molecular Probes, u.a.) zur absoluten Quantifizierung eingesetzt werden. Die Färbung ist dann in Anlehnung an die Angaben des Herstellers des jeweiligen Produktes anzupassen. Basierend auf der bekannten Konzentration eingesetzter Beads erfolgt bei der Auswertung der Messung die Ermittlung der absoluten Zellzahlen.

Die Benutzung eines Durchflusszytometers mit der Möglichkeit zum True Volumetric Counting (Partec) erlaubt den Verzicht auf Beads.

Zur Kosteneinsparung kann alternativ bei anderen Durchflusszytometern auch die Flussratenkalibrierung zum Einsatz kommen.

Die Messdauer kann abhängig von der Flussrate des verwendeten Durchflusszytometers sowie dem in der Probe vorhandenen Leukozytengehalt an die jeweils erforderliche Genauigkeit der Untersuchung angepasst werden. Die Einstellungen am Durchflusszytometer können dabei z.B. so gewählt werden, dass immer so lange gemessen wird, bis eine bestimmte Anzahl an Beads oder an Leukozyten an Ereignissen einer speziellen Leukozytensubpopulation detektiert wurde. Beim Einsatz der Flussratenkalibrierung wird vorzugsweise eine bestimmte Messdauer definiert.

### Beispiel 3: Bestimmung von Hühnerblut

Im Folgenden wird die Durchführung einer Blutbildbestimmung beim Huhn unter Verwendung eines Panleukozytenmarkers, eines Thrombozytenmarkers sowie von Markern für spezifische Leukozytensubpopulation beschrieben.

Insbesondere bei der Färbung aufgereinigter PBLs können durch die Kombination eines primären aCD45 und eines sekundären fluorochromgekoppelten αIgG die drei Populationen Erythrozyten, Thrombozyten und Leukozyten unterschieden werden. Bei der Färbung von Vollblut und der Verwendung von Direktkonjugaten kann es beim alleinigen Einsatz eines Panleukozytenmarkers aber zu Überschneidungen der Thrombozytenpopulation mit den Leukozyten und der Erythrozyten mit den Thrombozyten kommen, so dass die einzelnen Populationen nicht sicher abgegrenzt bzw. quantifiziert werden können.

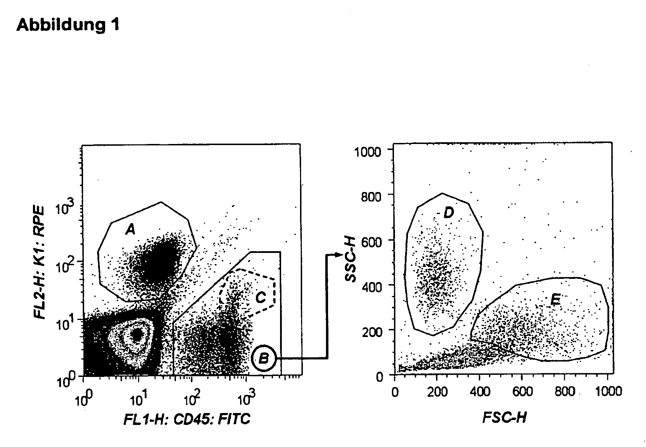

Durch die CD45-FITC/K1 RPE-Färbung (Abbildung 1) gelingt eine sichere Darstellung von Thrombozyten (A) und Leukozyten (B). Im FSC-H/SSC-H-Dotplot ist bereits eine teilweise Differenzierung der Leukozyten (B) möglich. Dabei zeigen sich die Heterophilen Granulozyten als eine eindeutig abgrenzbare Population (D). Da sich Monozyten schwach durch K1 anfärben lassen, können diese bereits in der FL1-H/FL2-H-Darstellung als Population (C) erkannt werden. Noch besser zeichnen sich die Monozyten als Population (E) bei der Darstellung aller Leukozyten (B) im FSC-H/SSC-H-Plot ab.

Unter Verwendung zusätzlicher Marker für Leukozytensubpopulationen, z.B. Antikörper gegen CD4 zum Nachweis von T-Helferzellen, Antikörper gegen CD8α zum Nachweis von cytotoxischen T-Zellen, Antikörper gegen den γδ-T-Zellrezeptor (TCRγδ) zur Bestimmung von γδ-T-Zellen oder Antikörpern gegen BU1 zur Bestimmung von B-Zellen, können durch das erfindungsgemäße Verfahren Subpopulationen von Leukozyten eindeutig differenziert werden.

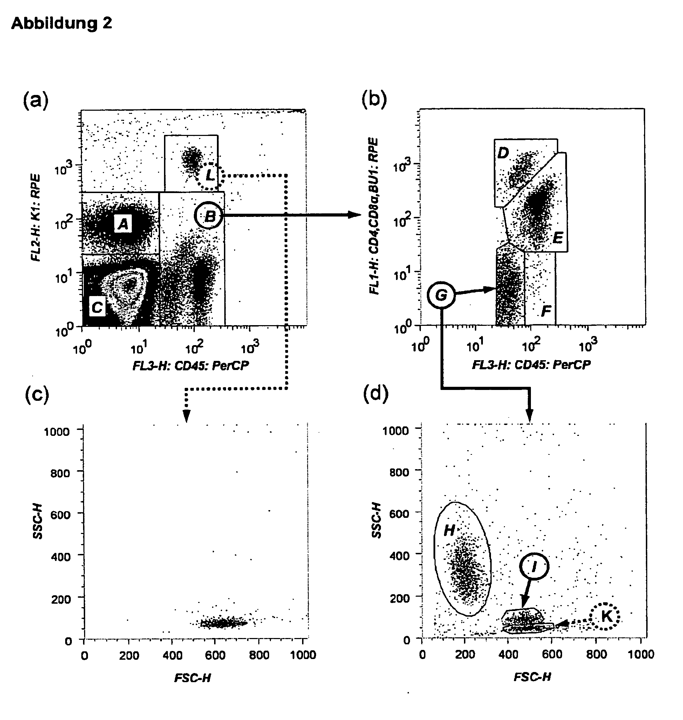

Abbildung 2 zeigt die Auswertung einer mit αCD45PerCP, K1RPE, KUL01RPE, αCD4FITC, αCD8αFITC, BU1FITC und αTCRγδFITC gefärbten Hühnerblutprobe. In der FL3-H/FL2-H-Darstellung (a) ist die Thrombozytenpopulation (A) eindeutig abgegrenzt. Die CD45PerCP hochpositiven Monozyten (L) kommen durch die KUL0RPE-Färbung extrem weit im FL2-H-positiven Bereich zu liegen. In dieser Darstellung zeichnen sich bereits die weiteren Subpopulationen der Leukozyten (B) ab. Besser gelingt die Differenzierung durch eine Übertragung in ein FL3-H/FL1-H-Plot (b). Die B-Lymphozyten (D) und T-Lmphozyten (E) lassen sich getrennt erfassen. Um die restlichen CD45PerCP mittel-positiven Leukozyten weiter zu klassifizieren, bietet sich die FSC-H/SSC-H-Darstellung (c) an. Hier lassen sich erneut drei Subpopulationen (H), (I) und (K) erkennen. Aufgrund des Vergleichs mit mikroskopisch erstellten Blutbildern sowie der hohen Granularität der Zellen konnte die Population (H) als die Heterophilen Granulozyten identifiziert werden. Bei den Populationen (I) und (K) handelt es sich vermutlich um die Eosinophilen und Basophilen Granulozyten. Die abschließende Identifikation dieser Zellpopulationen wird experimentell mit einem Zellsorter nachvollzogen werden.

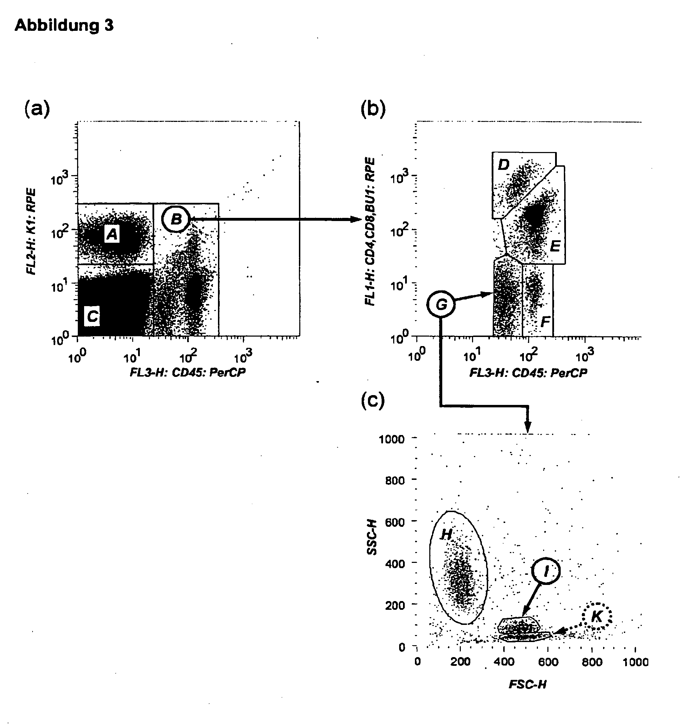

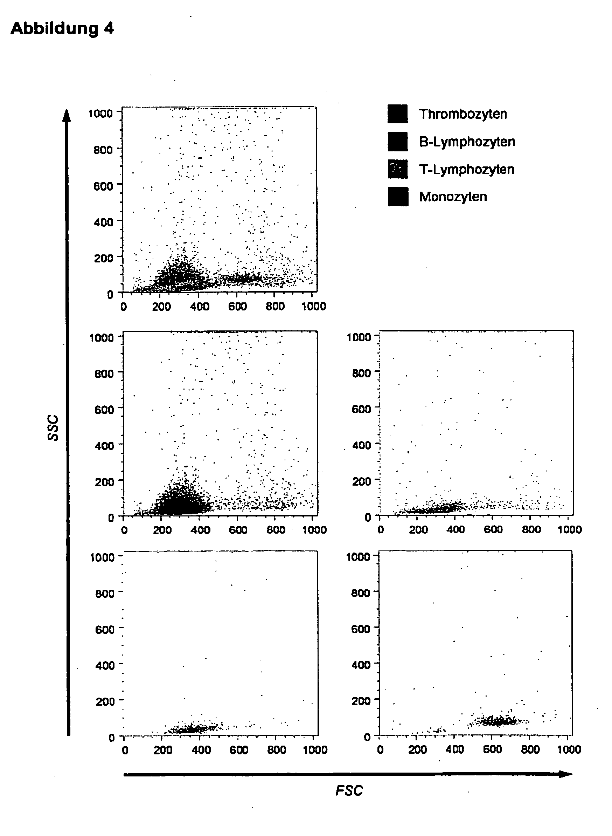

Ein ähnlich gutes Ergebnis lässt sich auch unter Verzicht auf den Einsatz des KUL01 zur Identifikation der Monozyten erzielen. Abbildung 3 zeigt die Auswertung der mit den Antikörpern αCD45PerCP, K1RPE, αCD4 FITC, αCD8aFITC, BU1FITC und αTCRγδFITC gefärbten Probe. Die Ergebnisse stimmen im Wesentlichen mit den Resultaten der an der gleichen Blutprobe durchgeführten, den Antikörper KUL01 einschließenden Färbung (Abbildung 2) überein. Der einzige wesentliche Unterschied besteht in der Identifikation der Monozyten. Sie befinden sich bei der KUL01-freien Färbung im gate F. Die gegenüber der Messung von Abbildung 2 optimaleren FSC-H/SSC-H-Einstellungen erlauben zudem die Identifikation der Monozyten (blau) aufgrund ihrer FSC-H/SSC-H-Charakteristika (Abbildung 4).

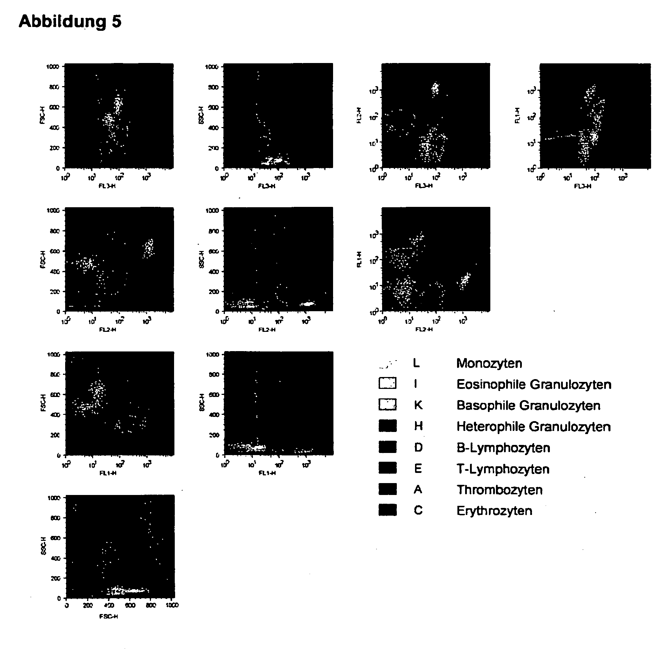

Zur umfassenderen Dokumentation der Charakteristika der verschiedenen identifizierbaren Zellpopulationen wurde eine Tafel (Abbildung 5) erstellt. Darin enthalten sind alle durch Permutation der Parameter FSC-H, SSC-H, FL1-H, FL2-H und FL3-H möglichen dotplot-Varianten der bereits aus Abbildung 2 bekannten Messung. Dass alle durch verschiedene Farben kenntlich gemachten Populationen in jeder möglichen Darstellung als natürlich wirkende Punktwolken in Erscheinung treten, demonstriert, dass die angewandte Färbetechnik reell existierende Zellpopulationen identifiziert, die einerseits in allen Parametern in sich homogen sind und sich andererseits untereinander gleichzeitig in einem oder mehreren Parametern unterscheiden.

Bei der hier dargestellten Antikörperkombination kommen verschiedene, mit dem gleichen Farbstoff gekennzeichnete Antikörper zum Einsatz.

Da sich aber z.B. die B-Lymphozyten stärker mit BU1FITC anfärben als die cytotoxischen T-Zellen mit αCD8α FITC, die T-Helferzellen mit αCD4 FITC und die γδ-T-Lymphozyten mit αTCRγδ FITC, können sie trotzdem von den anderen Lymphozyten unterschieden werden (Abbildung 2 Population (D), Abbildung 3 Population (D)). Dies wird außerdem dadurch erleichtert, dass die B-Lymphozyten sich schwächer als die anderen genannten Zelltypen mit αCD45 FITC anfärben.

Andererseits können mit dem Verfahren auch mehrere Zell-Populationen mit verschiedenen Markern, die aber mit dem gleichen Farbstoff gekoppelt sind, als eine Gruppe dargestellt werden. Bei der hier aufgezeigten Antikörperkombination erfolgt dies für die Summe der T-Lymphozyten, deren Subpopulationen durch die Antikörper αCD4, αCD8, αTCRγδ markiert werden (Abbildung 2 Population (E), Abbildung 3 Population (E)).

Die Identifikation der verschiedenen Zellpopulationen bei der graphischen Auswertung der Messdaten erfordert die Anwendung von "Gatingstrategien". Das Gating kann manuell bei der Auswertung der Messdaten mit Hilfe einer Software zur Verarbeitung durchflusszytometrischer Daten vorgenommen werden. Alternativ kann dies aber auch in Form einer automatisierten Datenalyse erfolgen.

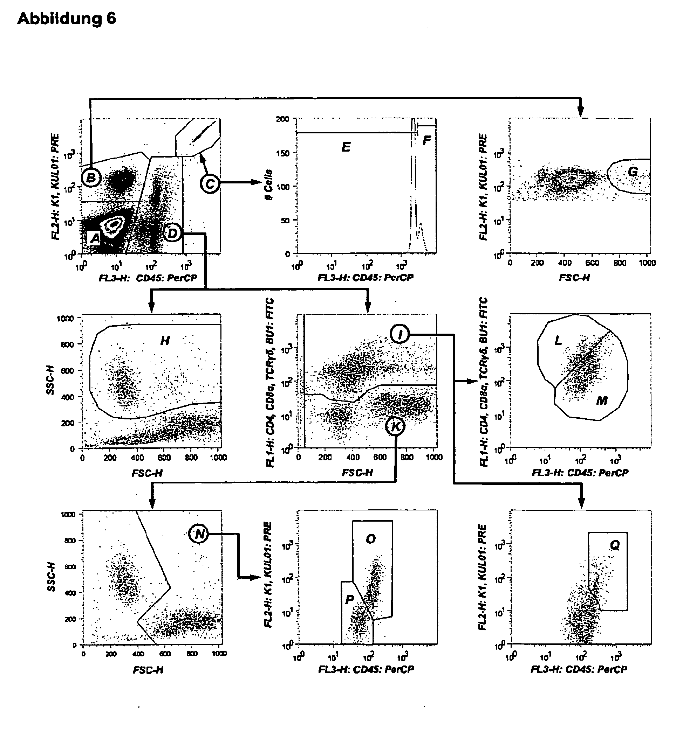

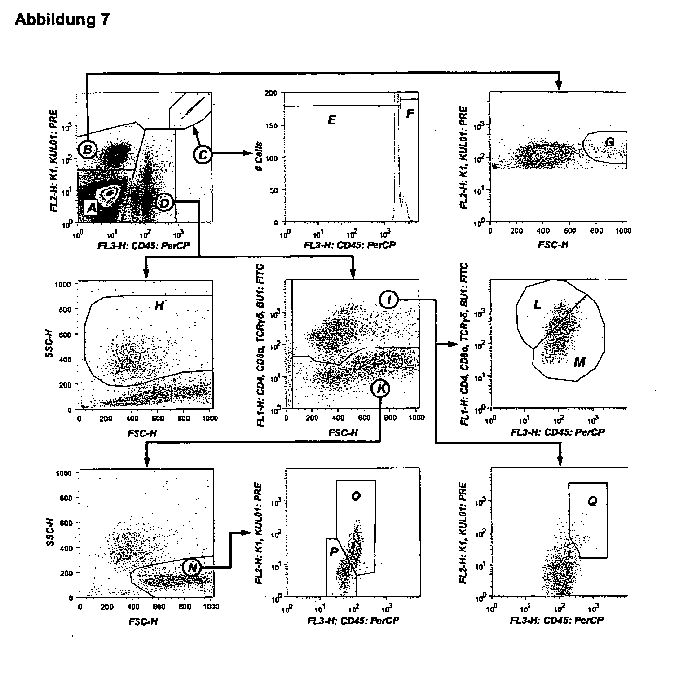

Abbildung 6 zeigt eine Gatingstrategie für vor der Färbung nicht fixierte Blutproben. Sie wurde so optimiert, dass auch frisch fixierte Proben (Abbildung 7) mit ihr analysiert werden können. In der FL3-H/FL2-H-Darstellung werden Erythrozyten (A), Thrombozyten (B), Beads (C) und Leukozyten (D) als Populationen erfasst. Bei Bedarf werden die Thrombozyten (B) in ein FSC-H/FL2-H dotplot übertragen, um die Probe auf einen erhöhten Gehalt an Thrombozytenaggregaten (Population G) zu überprüfen. Dieser kann auf eine fehlerhafte Blutentnahme hindeuten. Die Leukozyten (D) werden in zwei verschiedene Darstellungen übertragen. Im FSC-H/SSC-H-dotplot werden die Heterophilen Granulozyten als Population (H) erfasst. Im FSC-H/FL1-H-dotplot werden die Populationen (I) und (K) voneinander getrennt. Die Darstellung von (I) im FL3-H/FL1-H-Plot erlaubt die Differenzierung von B-Lymphozyten (L) und T-Lymphozyten (M). Im FL3-H/FL2-H-Plot können aus der Population (I) auch die Lymphozyten-Monozyten-Aggregate erfasst werden. Aus der Population (K) werden im FSC-H/SSC-H-Plot die Granulozyten entfernt. Die verbleibenden Zellen (N) lassen sich im FL3-H/FL2-H-Plot in Monozyten (O) und restliche Leukozyten (P) (Basophile Granulozyten, Eosinophile Granulozyten) aufteilen. Die Beads (C) werden im FL3-H-Histogramm auf den Prozentsatz an Aggregaten (F) untersucht.

Im Wesentlichen können frisch fixierte, innerhalb weniger Stunden gemessene Proben nach dem eben geschilderten Schema ausgewertet werden. Lediglich Gate N sollte etwas anders gelegt werden.

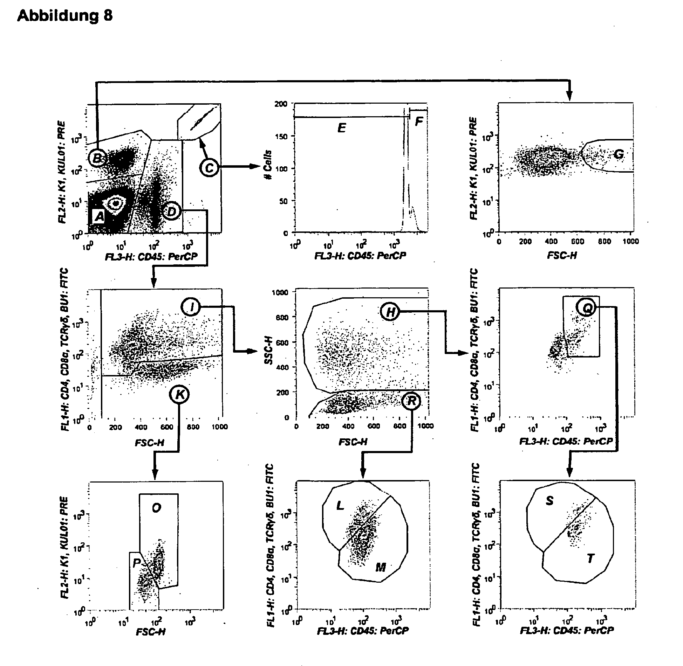

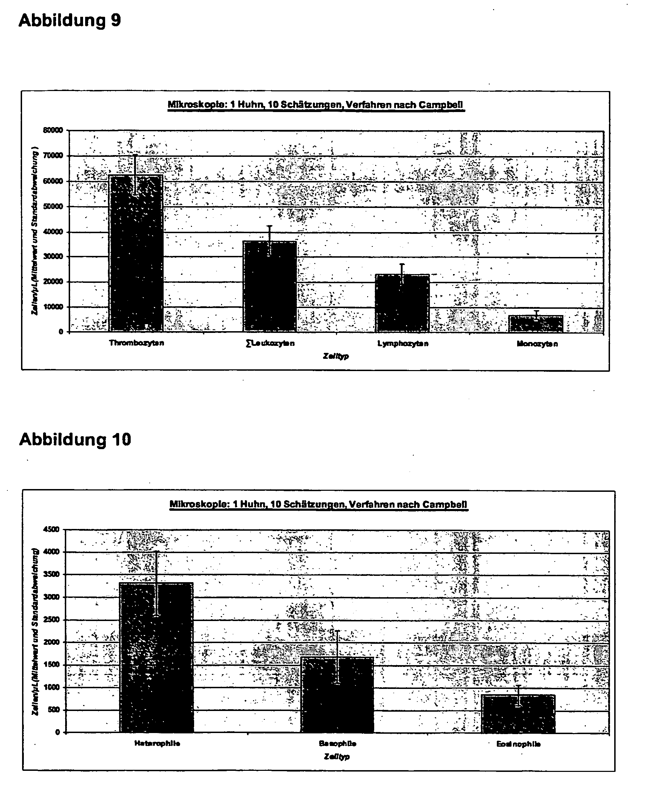

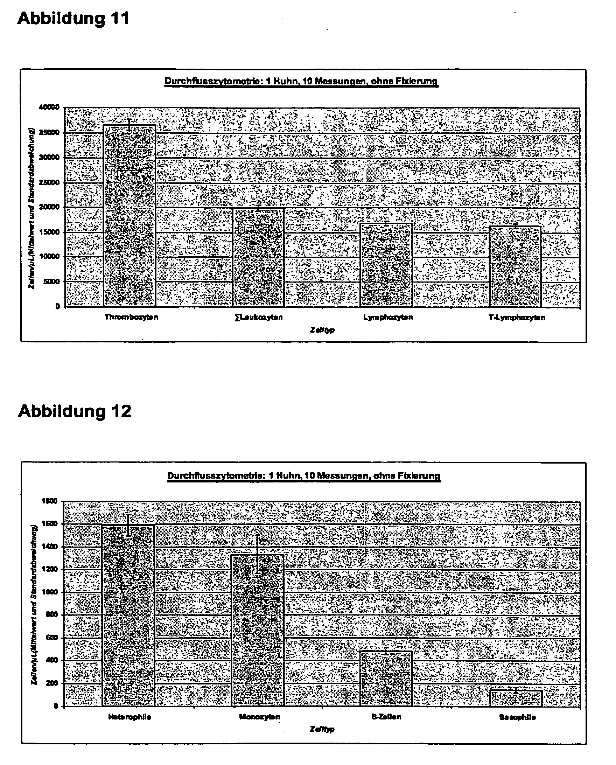

Werden die Proben am dritten Tag nach der Fixierung gemessen, sollte das Gatingprotokoll adaptiert werden (Abbildung 8). Da die Heterophilen Granulozyten zu diesem Zeitpunkt eine FL1-H Fluoreszenz entwickelt haben, werden diese nun aus der Population (I) im FSC-H/SSC-H-Plot als Population (H) identifiziert. Aus der verbleibenden Population (R) können im FL3-H/FL1-H-Plot B-Lymphozyten (L) und T-Lymphozyten (M) erfasst werden. Die Population (K) lässt sich im FSC-H/FL2-H-Plot in Restleukozyten und Monozyten auftrennen. Nach Übertragung der Population (H) in ein FL3-H/FL1-H-Plot können Aggregate (Q) aus Heterophilen Granulozyten und Lymphozyten dargestellt werden. Im FL-3H/FL1-H-Plot lassen sich dabei Aggregate mit T-Lymphozyten(T) und B-Lymphozyten (S) unterscheiden.

Alternativ zu der in Tabelle 1 aufgeführten Färbung können andere Antikörper eingesetzt werden.

Die bereits genannten monoklonalen Antikörper lassen sich durch äquivalente Marker ersetzen, die gegen die gleichen Antigene gerichtet sind oder zumindest die gleichen Populationen anfärben.
z.B.
Klon CT4 durch Klon 3-298,
K1 durch αCD41/CD61 (Klon 11C3) oder αCD51/61 (Klon 23C6).
KUL01 durch αgalectin-8 Klone.

Andere Antikörper lassen sich sogar durch Marker ersetzen, die andere Populationen darstellen:
z.B. kann die Kombination αTCRγδ, αCD8 und αCD4 durch αCD3 ersetzt werden um die Summe der T-Lymphozyten zu markieren. Als weitere Alternative kann eine Kombination aus, TCRγδ, TCRαβ(Vβ₁) und TCRαβ(Vβ₂) zum Einsatz kommen. Beide Alternativen führen allerdings zu einer etwas schwächeren Färbung. Auch die Kombination aus αCD28(Klon 2-4) und TCRγδ könnte alternativ zum Einsatz kommen. Weitere für das Verfahren nutzbare Lymphozytenmarker sind αCD5-Klone wie 2-191 sowie αCD6-Klone wie S3 und αCD11a Klone wie HUH73A (erhältlich bei WSU Monoclonal Antibody Center).

Bei Verwendung eines Durchflusszytometers mit 2 oder mehr Lasern lässt sich die Färbung erweitern. So kann z.B. bei Verwendung eines im vierten Fluoreszenzkanal detektierten Konjugates eine zusätzliche Zellpopulation getrennt erfasst werden. Durch Einsatz modernster Durchflusszytometer können bis zu 18 verschiedene Fluoreszfarbsstoffe eingesetzt und damit eine Vielzahl verscheidenster Zellpopulationen zur Darstellung gebracht werden. Beim Einsatz eines solchen Gerätes könnten z.B. die verschiedenen Lymphozytenpopulationen genauer charakterisiert und quantifiziert werden.

Für einige der verfügbaren Marker ist bekannt, dass sie mit den entsprechenden Antigenen anderer Vogelspezies kreuzreagieren:
Kreuzreaktivität mit Zellen von Präriehühnern bzw. Truthühnern wurde beschrieben für die αCD8-Klone 3-292, 3-298 und EP72 sowie für die αCD4-Klone 2-35, 7-125 und CT4 sowie für K1 und K55. K1 regiert auch mit Zellen von Enten und 23C6 detektiert sogar humane Zellen. Der αCD11a Klon HUH73A zeigte Kreuzreaktivität mit vielen verschiedenen Spezies. Mit einigen der bereits genannten Antikörpern dürfte zumindest bei der Pute ein weißes Blutbild erstellt werden können. Eine Bestimmung der Gesamtleukozyten- und Gesamtthrombozytenzahl mit teilweiser Leukozytendifferenzierung könnte mit Hilfe der Klone K1 und 23C6 auch bei weiter entfernten Spezies möglich sein, sofern einer der bekannten Panleukozytenmarker kreuzreagiert bzw. neu hergestellt wird.

Durch den kombinierten Einsatz eines Panleukozytenmarkers (z.B. 16-6, LT40 oder K55) und eines Thrombozytenmarkers (K1, αCD41/CD61, αCD51/61) wird aber auch bei der direkten Färbung von Vollblut eine sichere Abgrenzung der Populationen Erythrozyten, Thrombozyten und Leukozyten ermöglicht.

Im Vergleich mit der Mikroskopie (modifiziertes Schätzverfahren nach Campbell, Auszählung von 20 Sichtfeldern statt 5) konnte experimentell bereits die erheblich höhere Präzision des neuen durchflusszytometrischen Verfahrens gezeigt werden. Dafür wurden von einer Hühnerblutprobe 10 Ausstriche mikroskopisch vom gleichen Untersucher ausgezählt.

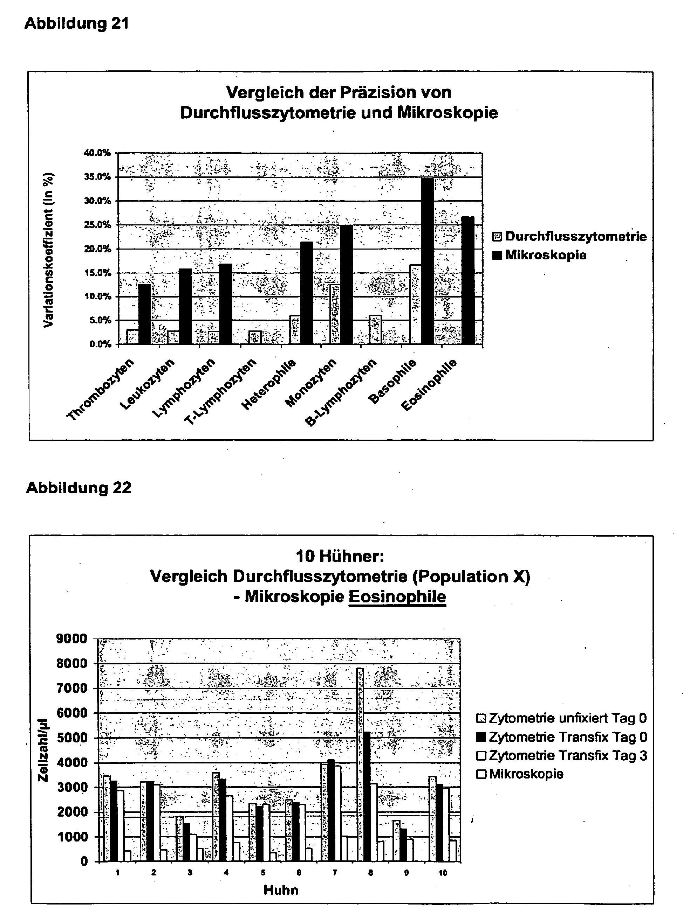

Eine andere Blutprobe wurde aliquotiert und 10 mal nach der neuen durchflusszytometrischen Technik aufbereitet und gemessen. Die Variationskoeffizienten (CV) der neuen Technik lagen wie erwartet weit unter denen der Mikroskopie (Tabellen 2 und 3). Da der Versuch noch nicht unter optimalen Bedingungen durchgeführt wurde, ist davon auszugehen, dass die Präzision des Verfahrens sogar noch deutlich gesteigert werden kann. Abbildung 21 zeigt einen Vergleich der Präzision von Mikroskopie und Durchflusszytometrie.

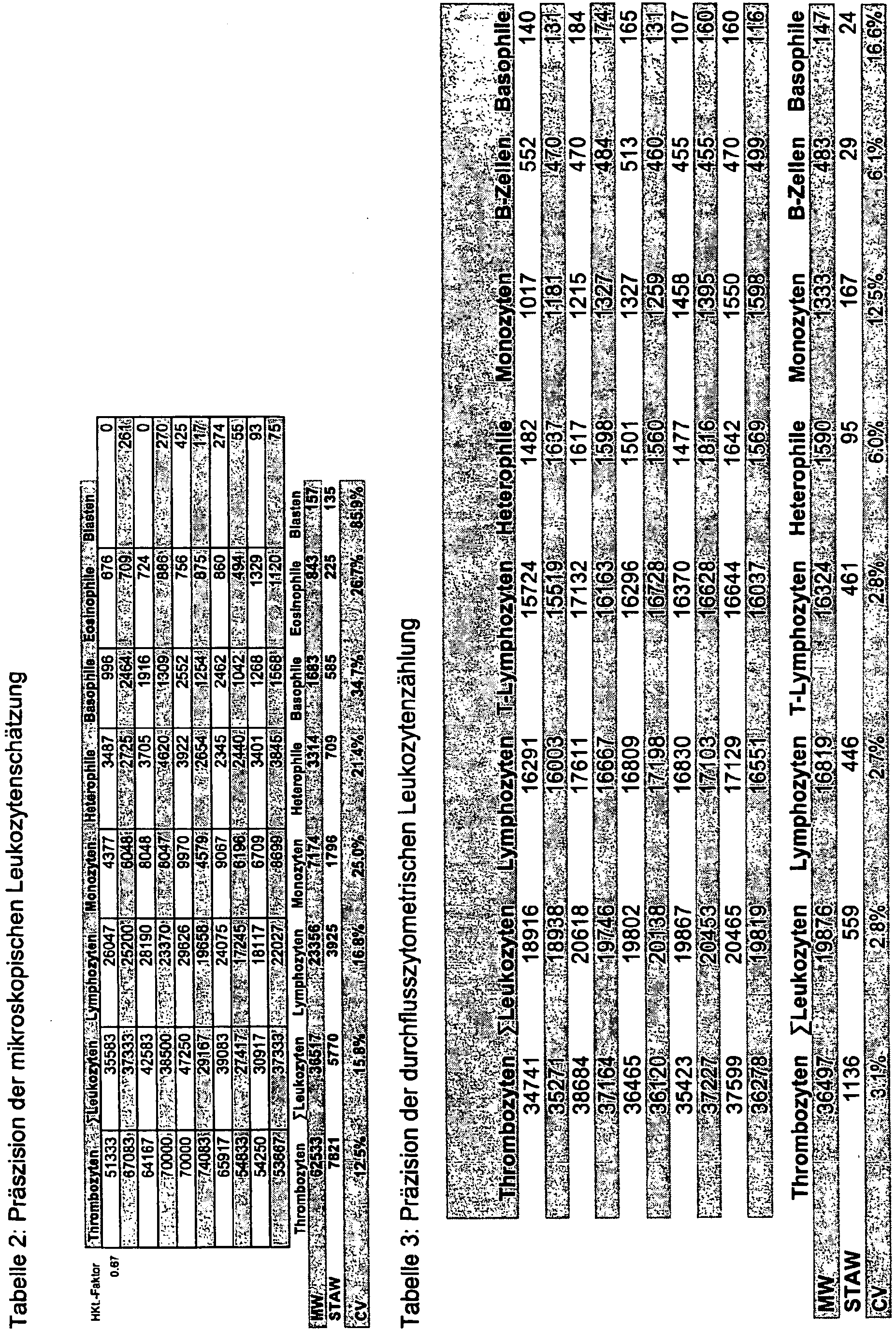

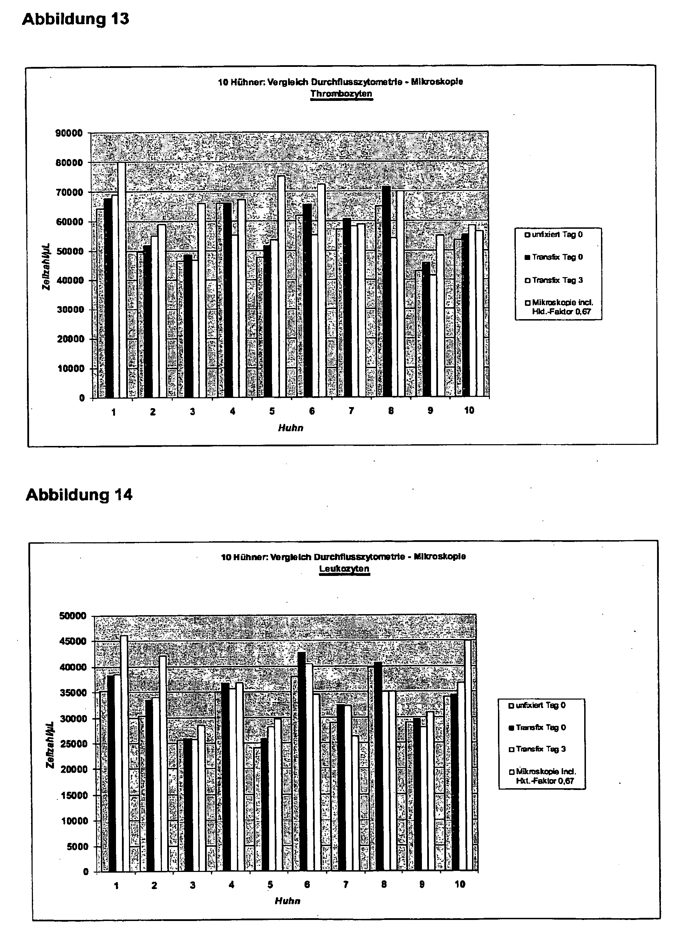

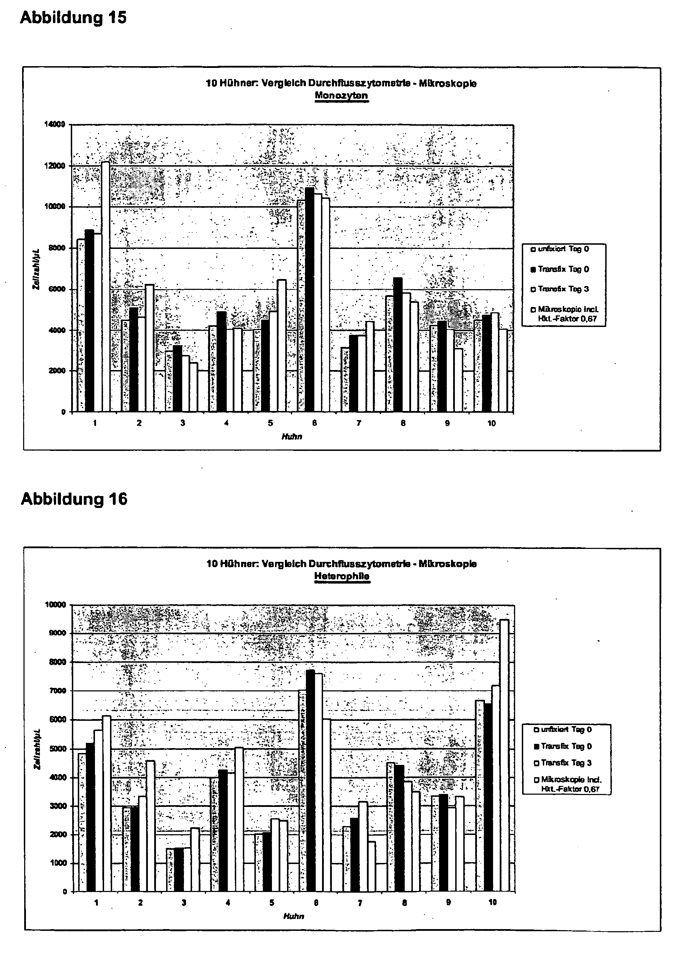

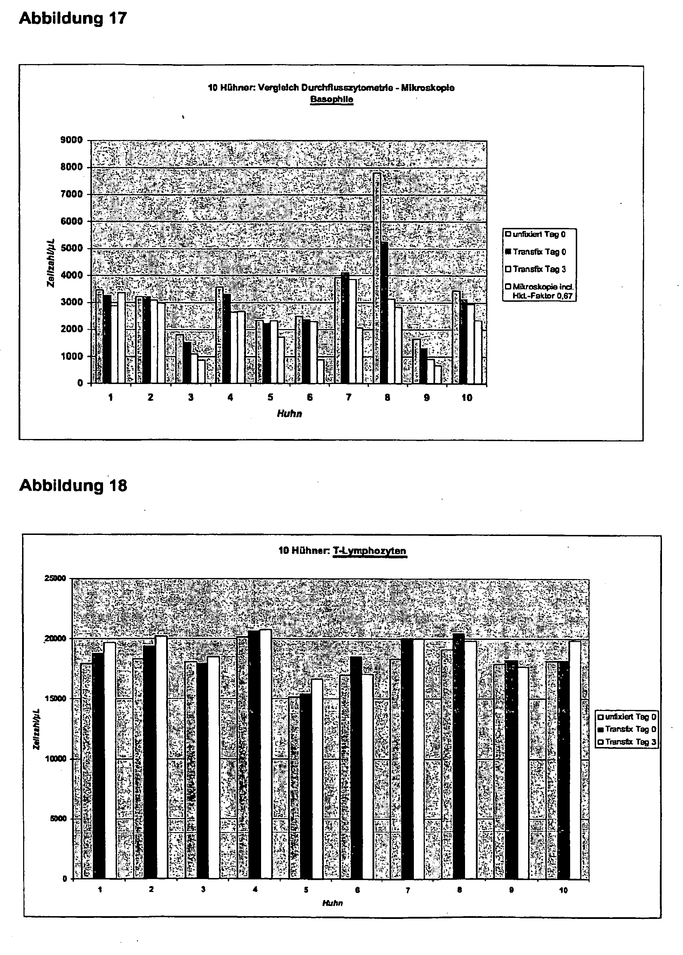

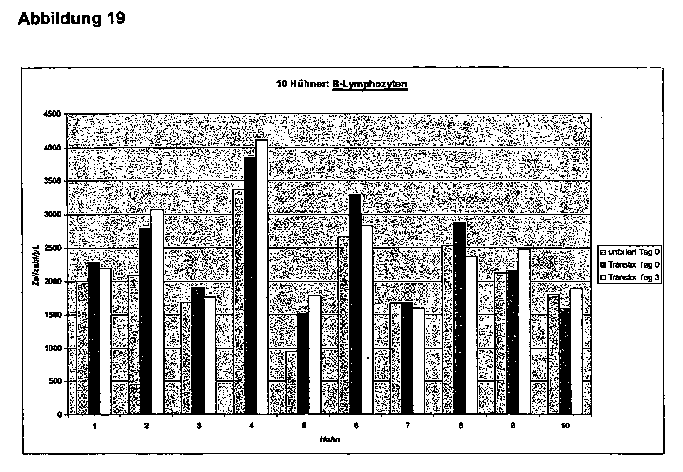

In einem weiteren Experiment wurden Blutproben von 10 Hühnern gewonnen und in einem Dreifachansatz untersucht. Ein Aliquot jeder Probe wurde bereits wenige Stunden nach der Entnahme gefärbt und analysiert. Ein anderer Teil der Probe wurde mit Transfix fixiert. Einige Stunden nach der Fixierung wurde ein Aliquot der fixierten Probe gefärbt und durchflusszytometrisch analysiert. Eine weitere Färbung mit anschließender Messung fand dann am dritten Tag nach der Entnahme/Fixierung statt. Zusätzlich wurde von jeder der 10 Blutproben ein Blutausstrich hergestellt und nach der Färbung mit dem Schätzverfahren nach Campbell untersucht. Über alle Proben hinweg zeigte sich bei dem Experiment für alle Zellpopulationen eine sehr gute Übereinstimmung zwischen den verschiedenen durchflusszytometrischen Messungen (unfixiert/ fixiert Tag 0/fixiert Tag 3) (Abbildungen 13-19 und 22). Die größeren Abweichungen zu den mit dem mikroskopischen Verfahren ermittelten Werten deuten auf dessen schlechtere Präzision hin.

### Beispiel 4: Erweiterungen des Verfahrens

Durch Einsatz von Markern gegen andere Zellen (Eosinophile Granulozyten, NK-Zellen etc.) kann die Leukozytendifferenzierung ausgeweitet werden. Die Grundlage dafür schafft das Verfahren durch die weitreichende Differenzierung der Leukozyten in der hier bereits dargestellten Form. Bei Verwendung eines Zellsorters und der Nutzung der oben beschriebenen Färbung können Zellpopulationen isoliert und zur weiteren Untersuchung (z.B. Genexpressionsanlysen), zum Anlegen von Zellkulturen bzw. zur Generierung spezifischer monoklonaler Antikörper genutzt werden. Da insbesondere PerCP bei verschiedenen Sortern aufgrund der Anfälligkeit für "photobleaching" nur bedingt eingesetzt werden kann, empfiehlt sich hier der Ersatz des αCD45-PerCP-Konjugates durch einen mit einem anderen Farbstoff gekoppelten Antikörper. Wenn bei der Zellsortierung keine absolute Quantifizierung erforderlich ist, bietet sich die Verwendung eines Biotin-markierten αCD45 an. In Verbindung mit diesem kann der am besten geeignete Farbstoff als Streptavidinkonjugat zum Einsatz kommen. Aufgrund der stärkeren Färbeintensität durch die indirekte Färbung ist dabei eine sehr deutliche CD45-Differenzierung zu erwarten, die wiederum ein gutes Zellseparierungsergebins ermöglicht.

Die hier beschriebene Technik kann auch mit anderen Farbstoffen, z.B. zur lebend/tot-Diskriminierung (Propidiumjodid, 7AAD, etc.) oder z.B. mit membranaffinen Farbstoffen DiOC₅(3), kombiniert werden. Insbesondere beim Fehlen entsprechender Panleukozytemarker für verschiedene Spezies könnte sich die Kombination eines Thrombozytenmarkers mit DiOC₅(3) zur Leukozytendarstellung als wertvoll erweisen.

Bei Verwendung eines modernen Durchflusszytometers wie dem CyAn ADP 7/9 Color (Beckman Coulter) mit Flussraten von bis zu 50 000 ev/s lässt sich die Messzeit im Vergleich zum FACScan (3500 ev/s) erheblich verkürzen. Dies verbessert die Wirtschaftlichkeit des Verfahrens bzw. erhöht bei Erreichen höherer Zellzahlen je Messung die Genauigkeit der Einzelmessung. Durch den Einsatz eines Autoloaders (als Karussell für Röhrchen oder im 96-Lochplattenformat) kann die Wirtschaftlichkeit des Verfahrens ebenfalls verbessert werden. Gleiches gilt für den Verzicht auf Beads zur absoluten Quantifizierung bei Benutzung eines Durchflusszytometers mit der Fähigkeit zum True Volumetric Counting bzw. Anwendung der Flussratenkalibrierung.

## Patentansprüche

1. Verfahren zur Bestimmung der Gesamtleukozytenzahl im Blut von Vögeln oder Hühnervögeln, umfassend die Schritte:
(a) Bereitstellung einer nicht-koagulierenden Blutprobe,
(b) gegebenenfalls Fixieren der Probe,
(c) Verdünnen der Probe,
(d) Inkontaktbringen der verdünnten Probe mit einem Panleukozytenmarker und einem Thrombozytenmarker,
(e) Messen der Probe in einem Durchflusszytometer ohne vorherige Wasch- oder Aufreinigungsschritte und
(f) quantitatives Bestimmen der Leukozytenpopulation sowie der Thrombozytenpopulation in der Probe.

2. Verfahren nach Anspruch 1,
**dadurch gekennzeichnet,**
**dass** mit ≥ 4mg/ml EDTA behandelte Vollblutproben bestimmt werden.

3. Verfahren nach einem der Ansprüche 1 oder 2,
**dadurch gekennzeichnet,**
**dass** der Panleukozytenmarker ausgewählt wird aus den Antikörpern anti-CD 45, 16-6, LT40 und K55 und dass der Thrombozytenmarker ausgewählt wird aus den Antikörpern K1, anti-CD41/CD61 und anti-CD51/CD61.

4. Verfahren nach einem der Ansprüche 1 bis 3,
**dadurch gekennzeichnet,**
**dass** in Schritt (e) weiterhin eine Vorwärts- und Seitwärts-Streuungs/Beugungsanalyse der Zellen durchgeführt wird.

5. Verfahren nach einem der Ansprüche 1 bis 4,
**dadurch gekennzeichnet,**
**dass** in Schritt (e) weiterhin eine Bestimmung von Leukozyten-Subpopulationen durchgeführt wird.

6. Verfahren nach Anspruch 5,
**dadurch gekennzeichnet,**
**dass** die Leukozyten-Subpopulationen ausgewählt werden aus
- Monozyten
- T-Helferzellen,
- cytotoxischen T-Zellen,
- γδ-T-Zellen,
- B-Zellen
- NK-Zellen
- Basophilen Granulozyten
- Eosinophilen Granulozyten
- Heterophilen/Neutrophile Granulozyten
- Stammzellen
und Kombinationen davon.

## Claims

1. A method for determining the total number of leukocytes in the blood of birds or galliformes, including the steps of:
(a) preparing a non-coagulating blood sample,
(b) where appropriate fixing the sample,
(c) diluting the sample,
(d) bringing the diluted sample into contact with a pan-leukocyte marker and a thrombocyte marker,
(e) measuring the sample in a flow cytometer without preceding washing or purifying steps, and
(f) quantitatively determining the leukocyte population and thrombocyte population in the sample.

2. A method according to Claim 1, **characterised in that** determination is performed on whole blood samples treated with ≥ 4 mg/ml of EDTA.

3. A method according to either of Claims 1 and 2, **characterised in that** the pan-leukocyte marker is selected from the antibodies anti-CD 45, 16-6, LT40 and K55, and **in that** the thrombocyte marker is selected from the antibodies K1, anti-CD41/CD61 and anti-CD51/CD61.

4. A method according to one of Claims 1 to 3, **characterised in that** in step (e) furthermore a forward and side scatter/diffraction analysis of the cells is performed.

5. A method according to one of Claims 1 to 4, **characterised in that** in step (e) furthermore a determination of leukocyte sub-populations is performed.

6. A method according to Claim 5, **characterised in that** the leukocyte sub-populations are selected from:
- monocytes
- T helper cells
- cytotoxic T cells
- γδ T cells
- B cells
- NK cells
- basophil granulocytes
- eosinophil granulocytes
- heterophil/neutrophil granulocytes
- stem cells
and combinations thereof.

## Revendications

1. Procédé pour déterminer le nombre de leucocyte total dans le sang d'oiseaux ou de galliformes, comprenant les étapes de :
(a) préparation d'un échantillon sanguin non coagulé,
(b) le cas échéant, fixation de l'échantillon,
(c) dilution de l'échantillon,
(d) mise en contact de l'échantillon dilué avec un marqueur pan-leucocytaire et un marqueur de thrombocyte,
(e) mesure de l'échantillon dans un cytomètre de flux sans étape préalable de lavage ou de purification, et
(f) détermination quantitative de la population de leucocytes, ainsi que de la population de thrombocytes dans l'échantillon.

2. Procédé selon la revendication 1, **caractérisé en ce que** l'on effectue l'analyse sur un échantillon de sang complet traité avec ≥ 4 mg/ml de EDTA.

3. Procédé selon la revendication 1 ou 2, **caractérisé en ce que** le marqueur pan-leucocytaire est choisi parmi les anticorps anti-CD45, 16-6, LT40 et K55 et **en ce que** le marqueur de thrombocytes est choisi parmi les anticorps K1, anti-CD41/CD61 et anti-CD51/CD61.

4. Procédé selon l'une des revendications 1 à 3, **caractérisé en ce que** dans l'étape (e), on réalise en outre, une analyse de diffusion/diffraction frontale et latérale des cellules.

5. Procédé selon l'une des revendications 1 à 4, **caractérisé en ce que** dans l'étape (e), on réalise en outre, une détermination des sous-populations de leucocyte.

6. Procédé selon la revendication 5, **caractérisé en ce que** les sous-populations de leucocyte sont choisies parmi
- les monocytes
- les cellules auxiliaires T
- les cellules T cytotoxiques,
- les cellules T γδ,
- les cellules B,
- les cellules NK,
- les granulocytes basophiles,
- les granulocytes éosinophiles,
- les granulocytes hétérophiles/neutrophiles,
- les cellules souches,
et leurs combinaisons.
